# EUROPEAN PATENT SPECIFICATION

(11) **EP 0 422 236 B1**
(45) Date of publication and mention of the grant of the patent: **14.06.1995**
(21) Application number: 90903956.2
(22) Date of filing: 06.03.1990
(51) Int. Cl.: C07C 45/65, C07C 49/796

(54) **PRODUCTION OF ALKENYLBENZOPHENONE**
VERFAHREN ZUR HERSTELLUNG VON ALKENYLBENZOPHENONEN
PROCEDE DE PRODUCTION D'ALCENYLBENZOPHENONE

(30) Priority: 06.03.1989 JP 53480/89
(43) Date of publication of application: 17.04.1991
(73) Proprietor: NIPPON PETROCHEMICALS COMPANY, LIMITED, Tokyo (JP)
(72) Inventor: SHIMIZU, Isoo, Yokohama-shi, Kanagawa 236 (JP); MATSUMURA, Yasuo, Yokohama-shi, Kanagawa 233 (JP); INOMATA, Yoshihisa, Kawasaki-shi, Kanagawa 210 (JP)
(74) Representative: Strehl Schübel-Hopf Groening & Partner
(86) International application number: JP9000289
(87) International publication number: WO9010615

(56) References cited:
- FR-A- 1 049 001
- JOURNAL OF ORGANIC CHEMISTRY OF THE USSR. vol. 24, no. 7, 1988, NEW YORK US pages 1346 - 1349; L.R. UVAROVA ET AL.: 'Aromatic Ketones with Terminal Vinyl Groups.'

## Description

### TECHNICAL FIELD

This invention relates to a method of producing alkenylbenzophenone. More particularly, the invention relates to a method of producing alkenylbenzophenone represented by the following formula (I) which is important as a polymerizable monomer or a raw material for the synthesis of medicines and perfumes. 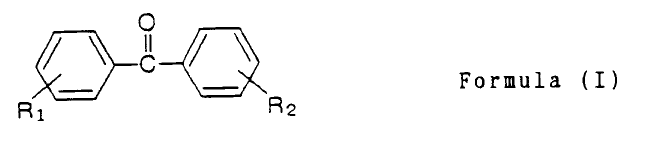
wherein R₁ is hydrogen, a methyl group or an ethyl group and R₂ is an ethenyl group or an isopropenyl group.

For example, 4-ethenylbenzophenone in which the alkenyl group is an ethenyl group is used as a polymerizable monomer for copolymerization. In addition, it is reported that the compound itself takes effect to relieve pain and inflammation. Furthermore, the compound is a useful intermediate material for preparing Ketoprofen (trade name) which takes large effects of pain-killing and anti-inflammation.

### BACKGROUND ART

Several methods for producing ethenylbenzophenone have been hitherto proposed. For example, there are following methods.
(1) 4-(1-Bromoethyl)benzophenone obtained by bromination of 4-ethylbenzophenone, is reacted in the presence of a base to obtain 4-ethenylbenzophenone (Polym. J. (Tokyo), Vol, 13, P. 521 (1981)). 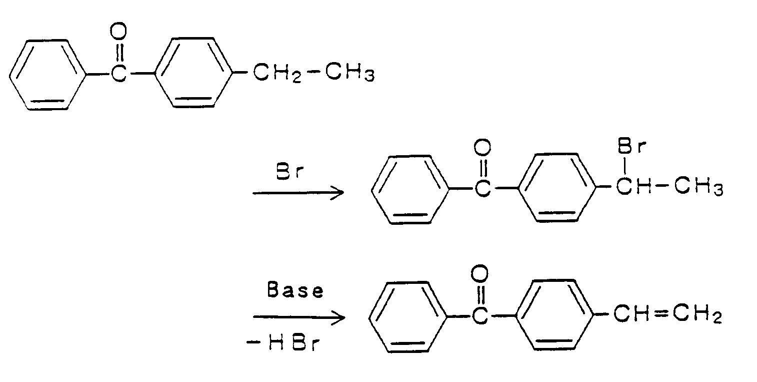
(2) 1-(3-Halophenyl)ethyl alcohol produced by the reduction of 3-haloacetophenone, is dehydrated to obtain 3-halostyrene. The thus obtained 3-halostyrene is allowed to react with metallic magnesium, which is followed by reaction with benzonitrile and then hydrolysis to obtain 3-ethenylbenzophenone (Japanese Laid-Open Patent Publication No. 56-26833. 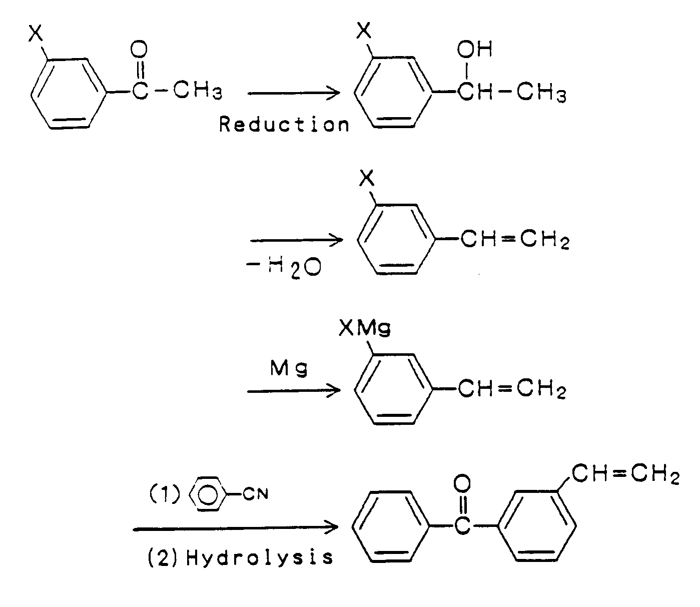 in the above formulae, X is halogen.

In the above method (1), the bromine used for the reaction is expensive and it is converted into bromide ion which is hardly recovered for reuse. Therefore, the cost for the bromination of 4-ethylbenzophenone is high, while the yield of reaction is not so high.

In the above method (2), the reaction using a Grignard reagent must be performed in a completely anhydrous state, which is not satisfactory in view of industrial production. Furthermore, the starting material 3-haloacetophenone is hardly available, in addition, the lowering of yield by the loss due to the polymerization of the ethenyl group in the steps of dehydration and hydrolysis cannot be avoided.

It cannot always be said that the above methods (1) and (2) are industrially desirable production methods, because of the above reasons as well as the fact that the numbers of production steps are large.

FR-A-1 049 007 discloses catalytic dehydrogenation of phenyl alkyl ketones having an alkyl substituent on the phenyl ring to give phenyl alkyl ketones with the corresponding alkenyl substituent on the phenyl ring.

In addition, a compound having an activated carbonyl group such as benzophenone generally reacts with hydrogen or a hydrogen donor without difficulty and the carbonyl group is hydrocracked. Accordingly, in the dehydrogenation of such a compound, the hydrogen produced by the dehydrogenation readily reacts with the carbonyl group and the aimed product cannot be obtained. Therefore, it cannot be considered as an industrial method.

It is known that 2-ethylnaphthalene can be obtained in good yield from 2-acetyl-5,6,7,8-tetrahydronaphthalene, in which acetyl group is supposed to be hydrocracked by the hydrogen that is liberated from naphthene ring in the presence of palladium catalyst (J. A. C. S., Vol. 65, P. 1097 (1943)). Furthermore, it is known that, in the case of benzophenone, the carbonyl group is readily hydrocracked in the presence of hydrogen donor even without any catalyst by the hydrogen liberated from the hydrogen donor and the benzophenone is converted into diphenylmethane (Fuel, Vol. 57, P. 650 (1978)). By the way, this result of experiment shows the fact that the carbonyl group of benzophenone is highly activated by two adjacent phenyl groups.

Accordingly, compounds having carbonyl groups which are hitherto known to be dehydrogenated, are only those having carbonyl groups connected to aliphatic groups such as methyl ethyl ketone, in which the carbonyl groups are inactive and hardly hydrocracked. Meanwhile, the vapor phase dehydrogenation by a dehydrogenation catalyst of benzophenone that is substituted with alkyl groups in which the carbonyl group is activated by two adjacent benzene rings, is not known.

In addition, as described above, it is known that benzophenone is hydrocracked by the produced hydrogen even without any catalyst to form diphenylmethane.

Accordingly, when alkyl group-substituted benzophenone is dehydrogenated, the formation of compounds having a diphenylmethane structure rather than the formation of alkenylbenzophenone is anticipated.

However, according to the investigation carried out by the present inventors with regard to the dehydrogenation of alkyl group-substituted benzophenone in the presence of a dehydrogenation catalyst, it was found out that the initially anticipated compound having a diphenyl methane structure is hardly produced but, to their surprise, alkenylbenzophenone is produced in a high selectivity by an elaborated method.

That is, the object of the present invention is to provide a novel method to synthesize alkenylbenzophenone easily and inexpensively in a high yield by a one step reaction process from readily available starting material.

### DISCLOSURE OF THE INVENTION

This invention relates to a novel method of producing alkenylbenzophenone represented by the formula (I), which method is characterized in that alkylbenzophenone represented by the Formula (II) is dehydrogenated in the presence of a dehydrogenation catalyst. 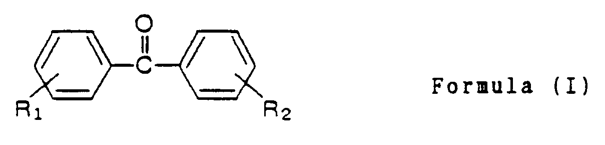
wherein R₁ is hydrogen, a methyl group or an ethyl group and R₂ is an ethenyl group or isopropenyl group. 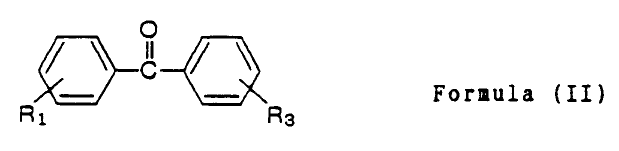
wherein R₁ is hydrogen, a methyl group or an ethyl group and R₃ is an ethyl group or isopropyl group.

In the present invention, the alkylbenzophenone represented by the above formula (II) is dehydrogenated in the presence of a dehydrogenation catalyst.

Exemplified as the alkylbenzophenone represented by the Formula (II) are 2-ethylbenzophenone, 3-ethylbenzophenone, 4-ethylbenzophenone, 2-isopropylbenzophenone, 4-isopropylbenzophenone and 4-methyl-4'-ethylbenzophenone.

In the dehydrogenation reaction of the present invention, hydrogen is liberated from the side chain having 2 to 3 carbon atoms and, corresponding to the above starting materials, the alkenylbenzophenone represented by the above formula (I) having a carbon-carbon double bond is obtained.

Exemplified as these compounds are 2-ethenylbenzophenone, 3-ethenylbenzophenone, 4-ethenylbenzophenone, 2-isopropenylbenzophenone, 4-isopropenylbenzophenone and 4-methyl-4'-ethenylbenzophenone.

According to the investigation carried out by the present inventors, for example, metal oxide-containing catalysts such as those hitherto used for the dehydrogenation of ethylbenzene to obtain styrene, can be used as dehydrogenation catalysts. Exemplified as such catalysts are chromia-alumina catalyst and iron oxide catalyst. As catalyst promoters, potassium, chromium, cerium, barium, bismuth, beryllium, molybdenum, vanadium, copper, magnesium, ruthenium, platinum, aluminium, nickel, cobalt, manganese, calcium, zinc, cesium and rubidium or their oxides can be used singly or in a mixture. Because these metal oxide catalysts are generally in solid state, they can be readily used in a fixed bed.

As to the pressure as a reaction condition for the dehydrogenation, the reaction can proceed faster under lower pressures in view of the equilibrium in dehydrogenation reaction and the reaction can proceed also faster at high temperatures because it is a strong endothermic reaction. Therefore, the reaction temperature is selected from the range of 450°C to 700°C, preferably 500°C to 650°C. Temperatures lower than 450°C are not desirable in practice because the dehydrogenation cannot proceed. On the other hand, a temperature above 700°C is not desirable either, because a side reaction such as cracking is caused to occur. The reaction pressure is set in the range of reduced pressure to 10 kg/cm², preferably from reduced pressure to 5 kg/cm². Furthermore, it is desirable to use excess steam as a heating medium. In order to improve the rate of reaction, the coexistence of molecular oxygen as a hydrogen acceptor can be allowed for the dehydrogenation.

The alkenylbenzophenone is dehydrogenated in vapor phase substantially. When occasion demands, a part of the material can be in liquid phase, however, complete vapor phase is desirable.

With regard to the factor relative to the reaction time, the LHSV is selected from the range of 0.001 to 100 hr⁻¹, preferably 0.01 to 10 hr⁻¹. When the LHSV value is lower than the above range, the yield is lowered due to the hydrocracking and polymerization of the reaction product. To the contrary, if the LHSV value exceeds the above range, this is not desirable because the yield is also lowered.

It should be noted that, in the method of the present invention, the vapor phase dehydrogenation must be carried out in a fixed bed flow reaction system. The reason for this is that, as described in the foregoing paragraphs, the carbonyl group of benzophenone is easily hydrocracked by the liberated hydrogen, and therefore the retention time in the reaction system must be made as short as possible. In the present invention, also in view of the prevention of the polymerization of the produced alkenylbenzophenone, the vapor phase dehydrogenation in a fixed bed is important. Accordingly, the liquid phase dehydrogenation and batchwise reaction are not desirable because their yields are not high.

In more preferable dehydrogenation conditions, the particle diameter of a dehydrogenation catalyst is 25% or less relative to the inner diameter of reaction tubes of a fixed bed reactor and the starting material of alkylbenzophenone is passed through the catalyst bed at 50 m/hr or higher in a linear velocity (on the basis of empty column, vapor phase, hereinafter referred to as "LV").

It is known that, when the diameter of catalyst is larger in view of the diameter of reaction tube, the inside wall of the reaction tube has an influence on the reaction. Furthermore, when the LV value exceeds a certain level, it has also an influence on the reaction. However, it cannot be anticipated at all that these influences are exerted markedly on a specific reaction like in the method of the present invention.

After the reaction, in order to avoid the side reaction such as the polymerization of alkenyl groups, the reaction mixture must be cooled and liquefied without delay. When steam is used as a heating medium, the separation of water is necessary.

The thus obtained dehydrogenation reaction mixture is then subjected to distillation, preferably reduced pressure distillation, to obtain alkenylbenzophenone.

Because the boiling point of the obtained alkenylbenzophenone is higher than the starting alkylbenzophenone, the former can be separated and recovered by distillation.

In the following, the present invention will be described in more detail with reference to examples.

### BEST METHOD FOR CARRYING OUT THE INVENTION

### Example 1

An iron oxide dehydrogenation catalyst (trademark: 64C made by Nissan Girdler Catalysts Co., Ltd.) of 0.5-1 mm in particle diameter was packed in a fixed bed flow reactor made of a stainless steel tube of 10 mm in inner diameter and 60 cm in length, thereby forming a catalyst bed of 20 cm in height. 4-Ethylbenzophenone and pure water in a weight ratio of 1:40 were preheated respectively to be vaporized, and they were mixed together and fed to the catalyst bed at a temperature of 570°C and an SV of 0.25. The reaction product was immediately cooled to room temperature and then liquefied. The organic layer obtained by gas/liquid separation was subjected to GC analysis. The result of the analysis was as follows:

| | |
|---|---|
| Conversion rate of 4-ethylbenzophenone | 58% |
| Selectivity of 4-ethenylbenzophenone | 61% |

### Example 2

Reaction was carried out in the like manner as in Example 1 except that 3-ethylbenzophenone was used in place of 4-ethylbenzophenone and the weight ratio of the material to be dehydrogenated to water was 1:20 to obtain 3-ethenylbenzophenone. The results thereof were as follows:

| | |
|---|---|
| Conversion rate of 3-ethylbenzophenone | 49% |
| Selectivity of 3-ethenylbenzophenone | 65% |

The organic layer was then distilled under reduced pressure to obtain a fraction of 145 to 150°C in distilling temperature (1 to 3 mm Hg vacuum). According to GC analysis, the purity of 3-ethenylbenzophenone was 92%.

### Example 3

Reaction was carried out in the like manner as in Example 1 except that 4-isopropylbenzophenone was used in place of 4-ethylbenzophenone and the reaction temperature was 550°C. The conversion rate and selectivity was 43% and 62%, respectively.

### Examples 4 and 5

Reaction was carried out in the like manner as in Example 1 except that an iron oxide dehydrogenation catalyst 64-EX (trademark, made also by Nissan Girdler Catalysts Co., Ltd.) and another iron oxide dehydrogenation catalyst containing magnesia which was prepared by the following procedure, were used. As the result, the values of (conversion rate of starting material)/(selectivity of corresponding alkenylbenzophenone) were 65%/43% and 53%/65%, respectively.

### - Preparation of Catalyst Containing Magnesia -

Ferric sulfate (550g) was dissolved in 3 liter of water and precipitate was removed. An aqueous solution of copper sulfate of 160g/500 ml was added thereto and it was poured into an aqueous suspension of magnesia of 958 g/8 lit. The precipitate was filtered off and washed with water, and it was suspended in 10 liter of water and 73 g/300 ml of potassium carbonate was added thereto. After drying, it was sintered at 650°C for 3 hours and molded by adding water.

### Example 6

Dehydrogenation of 4-ethylbenzophenone was carried out in the like manner as in Example 1 except that commercially available dehydrogenation catalyst of chromia-magnesia was used. As the result, 4-ethenylbenzophenone was obtained with a conversion rate of 4-ethylbenzophenone: 40% and selectivity of 4-ethenylbenzophenone: 50%.

### Example 7

Dehydrogenation was carried out in the like manner as in Example 1 except that reaction temperatures, LHSV values and other conditions were changed as shown in the following Table 1. The results thereof are shown also in Table 1.

**Table 1**

| Exper. | Reaction Temp. | LHSV | Dil.Rate with Water | *1 Conversion Rate | *2 Selectivity |
|---|---|---|---|---|---|
| No. 1 | 540 | 0.25 | 40 | 25% | 60% |
| No. 2 | 570 | 0.5 | 20 | 51% | 58% |
| No. 3 | 560 | 0.5 | 10 | 35% | 65% |

| | | | | | |
|---|---|---|---|---|---|
| (Notes) *1: Conversion rate of 4-ethylbenzophenone | | | | | |
| *2: Selectivity of 4-ethenylbenzophenone | | | | | |

### INDUSTRIAL APPLICABILITY

According to the method of the present invention, it is possible to produce alkenylbenzophenone in a high selectivity from alkyl group-substituted benzophenone by dehydrogenation in the presence of a dehydrogenation catalyst. In other words, alkenylbenzophenone can be produced by one step reaction process using readily available material, which process is easy, inexpensive and high in yield.

## Claims

1. A method of producing alkenylbenzophenone represented by the formula (I), which method is characterized in that alkylbenzophenone represented by the following formula (II) is dehydrogenated in the presence of a dehydrogenation catalyst, 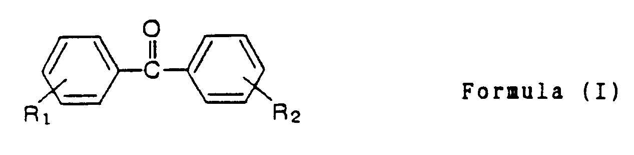 wherein R₁ is hydrogen, a methyl group or an ethyl group and R₂ is an ethenyl group or an isopropenyl group, 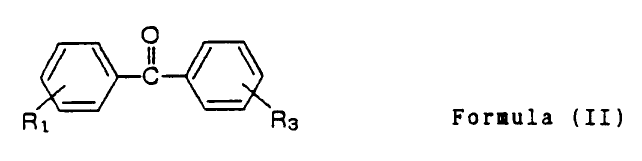 wherein R₁ is hydrogen, a methyl group or an ethyl group and R₃ is an ethyl group or an isopropyl group.

2. The method of producing alkenylbenzophenone as claimed in Claim 1, wherein the vapor phase dehydrogenation is carried out under the conditions of reaction temperatures of 450°C to 700°C and LHSV of 0.001 to 100 hr⁻¹ and the reaction product is cooled and liquefied immediately after the reaction.

## Patentansprüche

1. Verfahren zur Herstellung von Alkenylbenzophenon, dargestellt durch Formel (I), wobei das Verfahren dadurch gekennzeichnet ist, daß Alkylbenzophenon der folgenden Formel (II) in Gegenwart eines Dehydrierungskatalysators dehydriert wird, 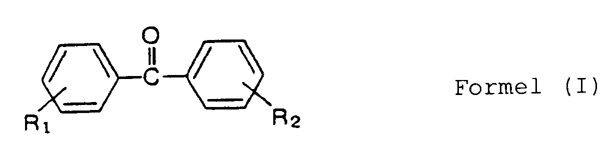 worin R₁ Wasserstoff, eine Methylgruppe oder eine Ethylgruppe und R₂ eine Ethenylgruppe oder eine Isopropenylgruppe bedeuten, 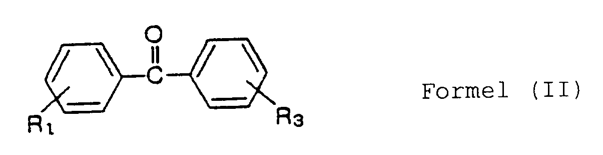 worin R₁ Wasserstoff, eine Methylgruppe oder eine Ethylgruppe und R₃ eine Ethylgruppe oder eine Isopropylgruppe bedeuten.

2. Verfahren zur Herstellung von Alkenylbenzophenon gemäß Anspruch 1 , wobei die Dampfphasen-Dehydrierung unter Bedingungen entsprechend Reaktionstemperaturen von 450°C bis 700°C und einer Stundenraumgeschwindigkeit (LHSV) von 0,001 bis 100 h⁻¹ durchgeführt wird und das Reaktionsprodukt unmittelbar nach der Reaktion abgekühlt und verflüssigt wird.

## Revendications

1. Procédé de préparation d'une alcénylbenzophénone représentée par la formule (I), qui se caractérise en ce qu'on déshydrogène une alkylbenzophénone représentée par la formule (II) en présence d'un catalyseur de déshydrogénation 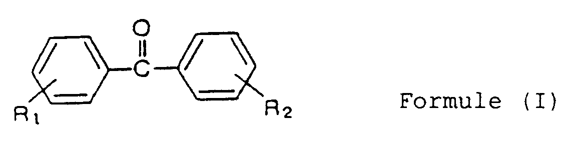 dans laquelle R₁ est un hydrogène, un groupe méthyle ou un groupe éthyle et R₂ est un groupe éthényle ou un groupe isopropényle 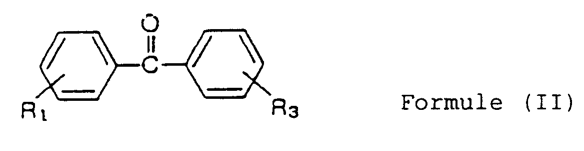 dans laquelle R₁ est un hydrogène, un groupe méthyle ou un groupe éthyle et R₃ est un groupe éthyle ou un groupe isopropyle.

2. Procédé de préparation d'alcénylbenzophénone selon la revendication 1, dans lequel la déshydrogénation en phase vapeur est conduite dans des conditions de température de réaction de 450°C à 700°C et une LHSV de 0,001 à 100 h⁻¹ et le produit de la réaction est refroidi et liquéfié immédiatement après la réaction.
